# EUROPEAN PATENT APPLICATION

(11) **EP 4 067 379 A1**
(43) Date of publication of application: **05.10.2022**
(21) Application number: 20893965.2
(22) Date of filing: 30.11.2020
(51) Int. Cl.: C07K 16/28, C07K 14/725, C07K 14/705, C12N 5/078, A61K 35/17, A61K 45/06, A61P 35/00

(54) **CHIMERIC ANTIGEN RECEPTOR SPECIFICALLY BINDING TO CD 300C ANTIGEN OR RECEPTOR THEREOF**

(30) Priority: 28.11.2019 KR 20190155027; 27.11.2020 KR 20200162200
(71) Applicant: CentricsBio, Inc., Seoul 05836 (KR)
(72) Inventor: JEON, Jaewon, Suwon-si, Gyeonggi-do 16512 (KR); LEE, Suin, Guri-si, Gyeonggi-do 11949 (KR); KIM, Haneul, Namyangju-si, Gyeonggi-do 12226 (KR)
(74) Representative: Dehns
(86) International application number: PCT/KR2020/017230
(87) International publication number: WO 2021/107726

(57) **Abstract**

The present disclosure relates to a chimeric antigen receptor that specifically binds to a CD300c antigen or a receptor thereof, immune cells expressing the same, and the like. The chimeric antigen receptor specifically binding to a CD300c antigen or a receptor thereof according to the present disclosure specifically recognizes cancer cells expressing the CD300c antigen or CD300c receptor and can directly effectively inhibit growth, metastasis, and the like of cancer. In addition, the chimeric antigen receptor activates T cells and the like in the body through inhibition of CD300c so that decreased adverse effects and increased cancer therapeutic effects are achieved. Thus, it is expected that the chimeric antigen receptor can be effectively used as an immunotherapeutic agent for various cancers.

<110>     CentricsBio

<120>     Chimeric antigen receptor specifically binding to CD300c antigen
          or its receptor

<130>     MPCT20-101

<150>     KR 10-2019-0155027
<151>     2019-11-28

<150>     KR 10-2020-0162200
<151>     2020-11-27

<160>     36

<170>     KoPatentIn 3.0

<210>     1
<211>     63
<212>     DNA
<213>     Artificial Sequence

<220>
<223>     CD8alpha Signal peptide


<400>     1
atggccttac cagtgaccgc cttgctcctg ccgctggcct tgctgctcca cgccgccagg          60

ccg                                                                         63


<210>     2
<211>     21
<212>     PRT
<213>     Artificial Sequence

<220>
<223>     CD8alpha Signal peptide


<400>     2
Met Ala Leu Pro Val Thr Ala Leu Leu Leu Pro Leu Ala Leu Leu Leu
  1               5                  10                  15

His Ala Ala Arg Pro
             20


<210>     3
<211>     714
<212>     DNA
<213>     Artificial Sequence

<220>
<223>     CK1 scFv


<400>     3
gaagtgcagc tgctggaaag tggaggtgga ctggtgcagc ctggcggcag cctgcgcctg          60

agctgtgccg ccagcggatt caccttcagc cgctatgcca tgacctgggt tcgccaagca         120

cctggcaaag gcctggaatg ggtgagcagc atgagcggca ccggcggcac cacctattat 180

gccgatagcg tgaaaggtcg ctttaccatc agccgcgata acagcaaaaa caccctgtat 240

ctgcagatga acagcctgcg cgccgaggac accgcagtct actactgtgc ccgcggcgcc 300

tatggctttg atcattgggg acaaggtact ctggtgaccg tgagcagcag tggaggaggt 360

agcggaggtg gtggatctgg aggtggaggt agtgaaatcg tgctgaccca gagccctggc 420

accctgagcc tgagccctgg cgaacgcgca acactgtcat gccgcgccag ccagagcatc 480

ggcaactatc tgaactggta tcagcagaaa ccaggtcagg ctccacgtct gctgatctat 540

gatgccagca acctggaaac cggcatccct gatcgcttct caggatctgg aagcggtacc 600

gattttaccc tgaccatcag ccgcctggaa cctgaggact ttgccgtgta ttattgtcag 660

cagagtagcg ccatccctta taccttcggt cagggcacta aagtggaaat caaa 714

<210> 4
<211> 238
<212> PRT
<213> Artificial Sequence

<220>
<223> CK1 scFv

<400> 4
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Ala Met Thr Trp Val Arg Gln Ala Pro Gly Lys Gly Leu Glu Trp Val
        35                  40                  45

Ser Ser Met Ser Gly Thr Gly Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Ala Tyr Gly Phe Asp His Trp Gly Gln Gly Thr Leu Val
            100                 105                 110

Thr Val Ser Ser Ser Gly Gly Gly Ser Gly Gly Gly Gly Ser Gly Gly
        115                 120                 125

Gly Gly Ser Glu Ile Val Leu Thr Gln Ser Pro Gly Thr Leu Ser Leu
    130                 135                 140

Ser Pro Gly Glu Arg Ala Thr Leu Ser Cys Arg Ala Ser Gln Ser Ile
145                 150                 155                 160

Gly Asn Tyr Leu Asn Trp Tyr Gln Gln Lys Pro Gly Gln Ala Pro Arg
                165                 170                 175

Leu Leu Ile Tyr Asp Ala Ser Asn Leu Glu Thr Gly Ile Pro Asp Arg
            180                 185                 190

Phe Ser Gly Ser Gly Ser Gly Thr Asp Phe Thr Leu Thr Ile Ser Arg
        195                 200                 205

Leu Glu Pro Glu Asp Phe Ala Val Tyr Tyr Cys Gln Gln Ser Ser Ala
    210                 215                 220

Ile Pro Tyr Thr Phe Gly Gln Gly Thr Lys Val Glu Ile Lys
225                 230                 235

<210>    5
<211>    729
<212>    DNA
<213>    Artificial Sequence

<220>
<223>    CL6 scFv

<400>    5
gaggtgcagc tgttggagtc tggtggaggc ttggtacagc ctggaggttc tcttcgcctc      60

tcctgtgcag cctccggatt cactttcagc agctacggta tgcattgggt cagacaggca     120

ccaggtaagg gactggagtg ggtctctgca attagcggta gcggtggtag cacttactac     180

gcagacagcg tgaagggtcg cttcaccatc tcacgcgaca actccaagaa caccctgtac     240

ctgcagatga acagccttcg cgcagaggac actgccgtgt attactgcgc agtcagtggt     300

gcaggtcgtg gtttcttcga ctactgggga caaggtactc tggtcactgt ctcctcaggt     360

ggaggcggtt caggcggagg tggatctggc ggtggcggat cccagtctgt gctgactcag     420

ccaccttcag catctggtac tccaggtcag cgcgtcacca tcagctgcag cggtagcagc     480

agcaacattg gtagcaacta cgtgtactgg tatcagcaac tcccaggcac cgctcctaag     540

ctcctgattt acgaggacaa caagcgtcct agtggtgtgc ctgatcgctt ttctgggtcc     600

aagtctggca cctcagcctc tctggctatc agtggacttc gctccgagga cgaggctgac     660

tattactgca gcagctacac tagcagcagc actgtgatct tcggcggtgg gaccaaactg     720

accgtccta                                                             729

<210>    6
<211>    243
<212>    PRT
<213>    Artificial Sequence

<220>
<223>    CL6 scFv

<400>    6
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Val Ser Gly Ala Gly Arg Gly Phe Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Gln Ser Val Leu Thr Gln Pro Pro Ser Ala
    130                 135                 140

Ser Gly Thr Pro Gly Gln Arg Val Thr Ile Ser Cys Ser Gly Ser Ser
145                 150                 155                 160

Ser Asn Ile Gly Ser Asn Tyr Val Tyr Trp Tyr Gln Gln Leu Pro Gly
                165                 170                 175

Thr Ala Pro Lys Leu Leu Ile Tyr Glu Asp Asn Lys Arg Pro Ser Gly
            180                 185                 190

Val Pro Asp Arg Phe Ser Gly Ser Lys Ser Gly Thr Ser Ala Ser Leu
        195                 200                 205

Ala Ile Ser Gly Leu Arg Ser Glu Asp Glu Ala Asp Tyr Tyr Cys Ser
    210                 215                 220

Ser Tyr Thr Ser Ser Ser Thr Val Ile Phe Gly Gly Gly Thr Lys Leu
225                 230                 235                 240

Thr Val Leu

<210>   7
<211>   720
<212>   DNA
<213>   Artificial Sequence

<220>
<223>   CL7 scFv

<400>   7
gaggtgcagc tgttggagtc tggtggaggc ttggtacagc ctggaggttc tcttcgcctc      60

tcctgtgcag cctccggatt cactttcagc cgctacgcaa tgagctgggt cagacaggca     120

ccaggtaagg gactggagtg ggtctctgca attagcggta gcggtggtag cacttactac     180

gcagacagcg tgaagggtcg cttcaccatc tcacgcgaca actccaagaa caccctgtac 240

ctgcagatga acagccttcg cgcagaggac actgccgtgt attactgcgc acgtagcagc 300

cagggtatct tcgacatctg gggacaaggt actctggtca ctgtctcctc aggtggaggc 360

ggttcaggcg gaggtggatc tggcggtggc ggatcccagt ctgtgctgac tcagccacct 420

tcagcatctg gtactccagg tcagcgcgtc accatcagct gcagtggtaa caatatcggt 480

actagacgcg tgcattggta tcagcaactc ccagacaccg ctcctaagct cctgatttac 540

agtaagaaca accgtcctag tggtgtgcct gatcgctttt ctgggtccaa gtctggcacc 600

tcagcctctc tggctatcag tggacttcgc tccgaggacg aggctgacta ttactgcgca 660

gcatgggacg acagcctgag cggtcctgtg ttcggcggtg ggaccaaact gaccgtccta 720

720

<210> 8
<211> 240
<212> PRT
<213> Artificial Sequence

<220>
<223> CL7 scFv

<400> 8
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Ser Gln Gly Ile Phe Asp Ile Trp Gly Gln Gly Thr Leu
            100                 105                 110

Val Thr Val Ser Ser Gly Gly Gly Gly Ser Gly Gly Gly Gly Ser Gly
        115                 120                 125

Gly Gly Gly Ser Gln Ser Val Leu Thr Gln Pro Pro Ser Ala Ser Gly
    130                 135                 140

Thr Pro Gly Gln Arg Val Thr Ile Ser Cys Ser Gly Asn Asn Ile Gly
145                 150                 155                 160

Thr Arg Arg Val His Trp Tyr Gln Gln Leu Pro Asp Thr Ala Pro Lys
                165                 170                 175

Leu Leu Ile Tyr Ser Lys Asn Asn Arg Pro Ser Gly Val Pro Asp Arg
            180                 185                 190

Phe Ser Gly Ser Lys Ser Gly Thr Ser Ala Ser Leu Ala Ile Ser Gly
        195                 200                 205

Leu Arg Ser Glu Asp Glu Ala Asp Tyr Tyr Cys Ala Ala Trp Asp Asp
    210                 215                 220

Ser Leu Ser Gly Pro Val Phe Gly Gly Gly Thr Lys Leu Thr Val Leu
225                 230                 235                 240

<210> 9
<211> 720
<212> DNA
<213> Artificial Sequence

<220>
<223> CL10 scFv

<400> 9
gaggtgcagc tgttggagtc tggtggaggc ttggtacagc ctggaggttc tcttcgcctc 60

tcctgtgcag cctccggatt cactttcagc agctacggta tgcattgggt cagacaggca 120

ccaggtaagg gactggagtg ggtctctgca attagcggta gcggtggtag cacttactac 180

gcagacagcg tgaagggtcg cttcaccatc tcacgcgaca actccaagaa caccctgtac 240

ctgcagatga acagccttcg cgcagaggac actgccgtgt attactgcgc aagcggttac 300

ggtctgatgg acgtgtgggg acaaggtact ctggtcactg tctcctcagg tggaggcggt 360

tcaggcggag gtggatctgg cggtggcgga tcccagtctg tgctgactca gccaccttca 420

gcatctggta ctccaggtca gcgcgtcacc atcagctgca ctcgtagcag cggtatcatc 480

gcaagcaact acgtgcagtg gtatcagcaa ctcccaggca ccgctcctaa gctcctgatt 540

taccgcaaca accagcgccc tagtggtgtg cctgatcgct tttctgggtc caagtctggc 600

acctcagcct ctctggctat cagtggactt cgctccgagg acgaggctga ctattactgc 660

agcagctacg caggtaacaa caacctggtg ttcggcggtg ggaccaaact gaccgtccta 720

720

<210> 10
<211> 240
<212> PRT
<213> Artificial Sequence

<220>
<223> CL10 scFv

<400> 10
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ser Gly Tyr Gly Leu Met Asp Val Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Gln Ser Val Leu Thr Gln Pro Pro Ser Ala Ser Gly Thr
    130                 135                 140

Pro Gly Gln Arg Val Thr Ile Ser Cys Thr Arg Ser Ser Gly Ile Ile
145                 150                 155                 160

Ala Ser Asn Tyr Val Gln Trp Tyr Gln Gln Leu Pro Gly Thr Ala Pro
                165                 170                 175

Lys Leu Leu Ile Tyr Arg Asn Asn Gln Arg Pro Ser Gly Val Pro Asp
            180                 185                 190

Arg Phe Ser Gly Ser Lys Ser Gly Thr Ser Ala Ser Leu Ala Ile Ser
        195                 200                 205

Gly Leu Arg Ser Glu Asp Glu Ala Asp Tyr Tyr Cys Ser Ser Tyr Ala
    210                 215                 220

Gly Asn Asn Asn Leu Val Phe Gly Gly Gly Thr Lys Leu Thr Val Leu
225                 230                 235                 240


<210>    11
<211>    717
<212>    DNA
<213>    Artificial Sequence

<220>
<223>    SL18 scFv


<400>    11
cgagtgcagc tgctggaaag tggaggtgga ctggtgcagc ctggcggcag cctgcgcctg        60

agctgtgccg ccagcggatt caccttcagc gattatcata tgcattgggt tcgccaagca       120

cctggcaaag gcctggaatg ggtgagcacc atcagcagca gcggcggcta tacctattat       180

gccgaaagcg tgaaaagccg ctttaccatc agccgcgata acagcaaaaa caccctgtat       240

ctgcagatga acagcctgcg cgccgaggac accgcagtct actactgtgc ccgatcgata       300

cgcctgcctc tggattattg gggacaaggt actctggtga ccgtgagcag cagtggagga 360

ggtagcggag gtggtggatc tggaggtgga ggtagtcaga gcgtgctgac ccagcctcct 420

agcgcctccg gtacaccagg acagcgcgtg actattagct gtagcggcaa caacatcggc 480

agcaaaggcg tgcattggta tcagcaactg cctggaactg cacctaagct gctgatctat 540

gaagatagca aacgccctag cggcgtgcgt gatcgcttta gcggtagcaa atcaggcacc 600

agcgccagcc tggccatcag cggccttcgc tccgaagatg aagccgatta ttattgtcag 660

agctatgata gcaccaaagg cgtggtgttt ggtggcggta ccaagctgac cgtgctg 717

<210> 12
<211> 239
<212> PRT
<213> Artificial Sequence

<220>
<223> SL18 scFv

<400> 12
Arg Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

His Met His Trp Val Arg Gln Ala Pro Gly Lys Gly Leu Glu Trp Val
        35                  40                  45

Ser Thr Ile Ser Ser Ser Gly Gly Tyr Thr Tyr Tyr Ala Glu Ser Val
    50                  55                  60

Lys Ser Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ser Ile Arg Leu Pro Leu Asp Tyr Trp Gly Gln Gly Thr Leu
            100                 105                 110

Val Thr Val Ser Ser Ser Gly Gly Gly Ser Gly Gly Gly Gly Ser Gly
        115                 120                 125

Gly Gly Gly Ser Gln Ser Val Leu Thr Gln Pro Pro Ser Ala Ser Gly
    130                 135                 140

Thr Pro Gly Gln Arg Val Thr Ile Ser Cys Ser Gly Asn Asn Ile Gly
145                 150                 155                 160

Ser Lys Gly Val His Trp Tyr Gln Gln Leu Pro Gly Thr Ala Pro Lys
                165                 170                 175

Leu Leu Ile Tyr Glu Asp Ser Lys Arg Pro Ser Gly Val Arg Asp Arg
            180                 185                 190

Phe Ser Gly Ser Lys Ser Gly Thr Ser Ala Ser Leu Ala Ile Ser Gly

195                 200                 205

Leu Arg Ser Glu Asp Glu Ala Asp Tyr Tyr Cys Gln Ser Tyr Asp Ser
    210                 215                 220

Thr Lys Gly Val Val Phe Gly Gly Gly Thr Lys Leu Thr Val Leu
225                 230                 235


<210>    13
<211>    489
<212>    DNA
<213>    Artificial Sequence

<220>
<223>    CD300c ECD


<400>    13
ggctattttc ctctgagcca ccccatgacc gtggcgggcc ccgtgggggg atccctgagt        60

gtgcagtgtc gctatgagaa ggaacacagg accctcaaca aattctggtg cagaccacca       120

cagattctcc gatgtgacaa gattgtggag accaaagggt cagcagggaa aaggaatggc       180

cgagtgtcca tcagggacag tcctgcaaac ctcagcttca cagtgaccct ggagaatctc       240

acagaggagg acgcaggcac ctactggtgt ggggtggata caccgtggct ccgagacttt       300

catgatccca ttgtcgaggt tgaggtgtcc gtgttcccgg ccgggacgac cacagcctcc       360

agcccccaga gctccatggg cacctcaggt cctcccacga agctgcccgt gcacacctgg       420

cccagcgtga ccagaaagga cagccccgaa cccagcccac accctggctc cctgttcagc       480

aatgtccgc                                                               489


<210>    14
<211>    163
<212>    PRT
<213>    Artificial Sequence

<220>
<223>    CD300c ECD


<400>    14
Gly Tyr Phe Pro Leu Ser His Pro Met Thr Val Ala Gly Pro Val Gly
1               5                   10                  15

Gly Ser Leu Ser Val Gln Cys Arg Tyr Glu Lys Glu His Arg Thr Leu
            20                  25                  30

Asn Lys Phe Trp Cys Arg Pro Pro Gln Ile Leu Arg Cys Asp Lys Ile
        35                  40                  45

Val Glu Thr Lys Gly Ser Ala Gly Lys Arg Asn Gly Arg Val Ser Ile
    50                  55                  60

Arg Asp Ser Pro Ala Asn Leu Ser Phe Thr Val Thr Leu Glu Asn Leu
65                  70                  75                  80

Thr Glu Glu Asp Ala Gly Thr Tyr Trp Cys Gly Val Asp Thr Pro Trp
                85                  90                  95

Leu Arg Asp Phe His Asp Pro Ile Val Glu Val Glu Val Ser Val Phe
            100                 105                 110

Pro Ala Gly Thr Thr Thr Ala Ser Ser Pro Gln Ser Ser Met Gly Thr
        115                 120                 125

Ser Gly Pro Pro Thr Lys Leu Pro Val His Thr Trp Pro Ser Val Thr
    130                 135                 140

Arg Lys Asp Ser Pro Glu Pro Ser Pro His Pro Gly Ser Leu Phe Ser
145                 150                 155                 160

Asn Val Arg

<210>    15
<211>    30
<212>    DNA
<213>    Artificial Sequence

<220>
<223>    GS linker

<400>    15
ggtggcggag gttctggagg tggaggttcc                                        30

<210>    16
<211>    10
<212>    PRT
<213>    Artificial Sequence

<220>
<223>    GS linker

<400>    16
Gly Gly Gly Gly Ser Gly Gly Gly Gly Ser
1               5                   10

<210>    17
<211>    135
<212>    DNA
<213>    Artificial Sequence

<220>
<223>    CD8 hinge

<400>    17
accacgacgc cagcgccgcg accaccaaca ccggcgccca ccatcgcgtc gcagcccctg       60

tccctgcgcc cagaggcgtg ccggccagcg gcggggggcg cagtgcacac gagggggctg      120

gacttcgcct gtgat                                                       135

<210>    18
<211>    45
<212>    PRT
<213>    Artificial Sequence

<220>
<223>    CD8 hinge


<400>    18
Thr Thr Thr Pro Ala Pro Arg Pro Pro Thr Pro Ala Pro Thr Ile Ala
1               5                   10                  15

Ser Gln Pro Leu Ser Leu Arg Pro Glu Ala Cys Arg Pro Ala Ala Gly
            20                  25                  30

Gly Ala Val His Thr Arg Gly Leu Asp Phe Ala Cys Asp
        35                  40                  45


<210>    19
<211>    81
<212>    DNA
<213>    Artificial Sequence

<220>
<223>    CD28 transmembrane domain


<400>    19
ttttgggtgc tggtggtggt tggtggagtc ctggcttgct atagcttgct agtaacagtg        60

gcctttatta ttttctgggt g                                                  81


<210>    20
<211>    27
<212>    PRT
<213>    Artificial Sequence

<220>
<223>    CD28 transmembrane domain


<400>    20
Phe Trp Val Leu Val Val Val Gly Gly Val Leu Ala Cys Tyr Ser Leu
1               5                   10                  15

Leu Val Thr Val Ala Phe Ile Ile Phe Trp Val
            20                  25


<210>    21
<211>    123
<212>    DNA
<213>    Artificial Sequence

<220>
<223>    CD28 intracellular domain


<400>    21
aggagtaaga ggagcaggct cctgcacagt gactacatga acatgactcc ccgccgcccc        60

gggcccaccc gcaagcatta ccagccctat gccccaccac gcgacttcgc agcctatcgc     120

tcc                                                                   123

<210>    22
<211>    41
<212>    PRT
<213>    Artificial Sequence

<220>
<223>    CD28 intracellular domain

<400>    22
Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210>    23
<211>    339
<212>    DNA
<213>    Artificial Sequence

<220>
<223>    CD3zeta intracellular domain

<400>    23
agagtgaagt tcagcaggag cgcagacgcc cccgcgtacc agcagggcca gaaccagctc      60

tataacgagc tcaatctagg acgaagagag gagtacgatg ttttggacaa gagacgtggc     120

cgggaccctg agatgggggg aaagccgaga aggaagaacc ctcaggaagg cctgtacaat     180

gaactgcaga aagataagat ggcggaggcc tacagtgaga ttgggatgaa aggcgagcgc     240

cggaggggca aggggcacga tggcctttac cagggtctca gtacagccac caaggacacc     300

tacgacgccc ttcacatgca ggccctgccc cctcgctaa                            339

<210>    24
<211>    112
<212>    PRT
<213>    Artificial Sequence

<220>
<223>    CD3zeta intracellular domain

<400>    24
Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr

20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>    25
<211>    1485
<212>    DNA
<213>    Artificial Sequence

<220>
<223>    CK1 CAR

<400>    25
atggccttac cagtgaccgc cttgctcctg ccgctggcct tgctgctcca cgccgccagg      60

ccggaagtgc agctgctgga aagtggaggt ggactggtgc agcctggcgg cagcctgcgc     120

ctgagctgtg ccgccagcgg attcaccttc agccgctatg ccatgacctg ggttcgccaa     180

gcacctggca aaggcctgga atgggtgagc agcatgagcg gcaccggcgg caccacctat     240

tatgccgata gcgtgaaagg tcgctttacc atcagccgcg ataacagcaa aaacaccctg     300

tatctgcaga tgaacagcct gcgcgccgag gacaccgcag tctactactg tgcccgcggc     360

gcctatggct ttgatcattg gggacaaggt actctggtga ccgtgagcag cagtggagga     420

ggtagcggag gtggtggatc tggaggtgga ggtagtgaaa tcgtgctgac ccagagccct     480

ggcaccctga gcctgagccc tggcgaacgc gcaacactgt catgccgcgc cagccagagc     540

atcggcaact atctgaactg gtatcagcag aaaccaggtc aggctccacg tctgctgatc     600

tatgatgcca gcaacctgga aaccggcatc cctgatcgct tctcaggatc tggaagcggt     660

accgatttta ccctgaccat cagccgcctg gaacctgagg actttgccgt gtattattgt     720

cagcagagta gcgccatccc ttataccttc ggtcagggca ctaaagtgga aatcaaaggt     780

ggcggaggtt ctggaggtgg aggttccacc acgacgccag cgccgcgacc accaacaccg     840

gcgcccacca tcgcgtcgca gcccctgtcc ctgcgcccag aggcgtgccg gccagcggcg     900

gggggcgcag tgcacacgag ggggctggac ttcgcctgtg atttttgggt gctggtggtg     960

gttggtggag tcctggcttg ctatagcttg ctagtaacag tggcctttat tattttctgg    1020

gtgaggagta agaggagcag gctcctgcac agtgactaca tgaacatgac tccccgccgc 1080

cccgggccca cccgcaagca ttaccagccc tatgccccac cacgcgactt cgcagcctat 1140

cgctccagag tgaagttcag caggagcgca gacgcccccg cgtaccagca gggccagaac 1200

cagctctata acgagctcaa tctaggacga agagaggagt acgatgtttt ggacaagaga 1260

cgtggccggg accctgagat ggggggaaag ccgagaagga agaaccctca ggaaggcctg 1320

tacaatgaac tgcagaaaga taagatggcg gaggcctaca gtgagattgg gatgaaaggc 1380

gagcgccgga ggggcaaggg gcacgatggc ctttaccagg gtctcagtac agccaccaag 1440

gacacctacg acgcccttca catgcaggcc ctgccccctc gctaa 1485

<210> 26
<211> 494
<212> PRT
<213> Artificial Sequence

<220>
<223> CK1 CAR

<400> 26
Met Ala Leu Pro Val Thr Ala Leu Leu Leu Pro Leu Ala Leu Leu Leu
1               5                   10                  15

His Ala Ala Arg Pro Glu Val Gln Leu Leu Glu Ser Gly Gly Gly Leu
            20                  25                  30

Val Gln Pro Gly Gly Ser Leu Arg Leu Ser Cys Ala Ala Ser Gly Phe
        35                  40                  45

Thr Phe Ser Arg Tyr Ala Met Thr Trp Val Arg Gln Ala Pro Gly Lys
    50                  55                  60

Gly Leu Glu Trp Val Ser Ser Met Ser Gly Thr Gly Gly Thr Thr Tyr
65                  70                  75                  80

Tyr Ala Asp Ser Val Lys Gly Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Arg Gly Ala Tyr Gly Phe Asp His Trp Gly
        115                 120                 125

Gln Gly Thr Leu Val Thr Val Ser Ser Ser Gly Gly Gly Ser Gly Gly
    130                 135                 140

Gly Gly Ser Gly Gly Gly Gly Ser Glu Ile Val Leu Thr Gln Ser Pro
145                 150                 155                 160

Gly Thr Leu Ser Leu Ser Pro Gly Glu Arg Ala Thr Leu Ser Cys Arg
                165                 170                 175

Ala Ser Gln Ser Ile Gly Asn Tyr Leu Asn Trp Tyr Gln Gln Lys Pro
            180                 185                 190

Gly Gln Ala Pro Arg Leu Leu Ile Tyr Asp Ala Ser Asn Leu Glu Thr
        195                 200                 205

Gly Ile Pro Asp Arg Phe Ser Gly Ser Gly Ser Gly Thr Asp Phe Thr
    210                 215                 220

Leu Thr Ile Ser Arg Leu Glu Pro Glu Asp Phe Ala Val Tyr Tyr Cys
225                 230                 235                 240

Gln Gln Ser Ser Ala Ile Pro Tyr Thr Phe Gly Gln Gly Thr Lys Val
                245                 250                 255

Glu Ile Lys Gly Gly Gly Gly Ser Gly Gly Gly Gly Ser Thr Thr Thr
            260                 265                 270

Pro Ala Pro Arg Pro Pro Thr Pro Ala Pro Thr Ile Ala Ser Gln Pro
        275                 280                 285

Leu Ser Leu Arg Pro Glu Ala Cys Arg Pro Ala Ala Gly Gly Ala Val
    290                 295                 300

His Thr Arg Gly Leu Asp Phe Ala Cys Asp Phe Trp Val Leu Val Val
305                 310                 315                 320

Val Gly Gly Val Leu Ala Cys Tyr Ser Leu Leu Val Thr Val Ala Phe
                325                 330                 335

Ile Ile Phe Trp Val Arg Ser Lys Arg Ser Arg Leu Leu His Ser Asp
            340                 345                 350

Tyr Met Asn Met Thr Pro Arg Arg Pro Gly Pro Thr Arg Lys His Tyr
        355                 360                 365

Gln Pro Tyr Ala Pro Pro Arg Asp Phe Ala Ala Tyr Arg Ser Arg Val
    370                 375                 380

Lys Phe Ser Arg Ser Ala Asp Ala Pro Ala Tyr Gln Gln Gly Gln Asn
385                 390                 395                 400

Gln Leu Tyr Asn Glu Leu Asn Leu Gly Arg Arg Glu Glu Tyr Asp Val
                405                 410                 415

Leu Asp Lys Arg Arg Gly Arg Asp Pro Glu Met Gly Gly Lys Pro Arg
            420                 425                 430

Arg Lys Asn Pro Gln Glu Gly Leu Tyr Asn Glu Leu Gln Lys Asp Lys
        435                 440                 445

Met Ala Glu Ala Tyr Ser Glu Ile Gly Met Lys Gly Glu Arg Arg Arg
    450                 455                 460

Gly Lys Gly His Asp Gly Leu Tyr Gln Gly Leu Ser Thr Ala Thr Lys
465                 470                 475                 480

Asp Thr Tyr Asp Ala Leu His Met Gln Ala Leu Pro Pro Arg
                485                 490


<210>   27
<211>   1500
<212>   DNA
<213>   Artificial Sequence

<220>
<223>    CL6 CAR

<400>    27
atggccttac cagtgaccgc cttgctcctg ccgctggcct tgctgctcca cgccgccagg      60

ccggaggtgc agctgttgga gtctggtgga ggcttggtac agcctggagg ttctcttcgc     120

ctctcctgtg cagcctccgg attcactttc agcagctacg gtatgcattg ggtcagacag     180

gcaccaggta agggactgga gtgggtctct gcaattagcg gtagcggtgg tagcacttac     240

tacgcagaca gcgtgaaggg tcgcttcacc atctcacgcg acaactccaa gaacaccctg     300

tacctgcaga tgaacagcct tcgcgcagag gacactgccg tgtattactg cgcagtcagt     360

ggtgcaggtc gtggtttctt cgactactgg ggacaaggta ctctggtcac tgtctcctca     420

ggtggaggcg gttcaggcgg aggtggatct ggcggtggcg gatcccagtc tgtgctgact     480

cagccacctt cagcatctgg tactccaggt cagcgcgtca ccatcagctg cagcggtagc     540

agcagcaaca ttggtagcaa ctacgtgtac tggtatcagc aactcccagg caccgctcct     600

aagctcctga tttacgagga caacaagcgt cctagtggtg tgcctgatcg cttttctggg     660

tccaagtctg gcacctcagc ctctctggct atcagtggac ttcgctccga ggacgaggct     720

gactattact gcagcagcta cactagcagc agcactgtga tcttcggcgg tgggaccaaa     780

ctgaccgtcc taggtggcgg aggttctgga ggtggaggtt ccaccacgac gccagcgccg     840

cgaccaccaa caccggcgcc caccatcgcg tcgcagcccc tgtccctgcg cccagaggcg     900

tgccggccag cggcgggggg cgcagtgcac acgagggggc tggacttcgc ctgtgatttt     960

tgggtgctgg tggtggttgg tggagtcctg gcttgctata gcttgctagt aacagtggcc    1020

tttattattt tctgggtgag gagtaagagg agcaggctcc tgcacagtga ctacatgaac    1080

atgactcccc gccgccccgg gcccacccgc aagcattacc agccctatgc cccaccacgc    1140

gacttcgcag cctatcgctc cagagtgaag ttcagcagga gcgcagacgc ccccgcgtac    1200

cagcagggcc agaaccagct ctataacgag ctcaatctag gacgaagaga ggagtacgat    1260

gttttggaca agagacgtgg ccgggaccct gagatggggg gaaagccgag aaggaagaac    1320

cctcaggaag gcctgtacaa tgaactgcag aaagataaga tggcggaggc ctacagtgag    1380

attgggatga aaggcgagcg ccggaggggc aaggggcacg atggccttta ccagggtctc    1440

agtacagcca ccaaggacac ctacgacgcc cttcacatgc aggccctgcc ccctcgctaa    1500

                                                                  1500

<210>    28
<211>    499
<212>    PRT
<213>    Artificial Sequence

<220>
<223>    CL6 CAR


<400>    28
Met Ala Leu Pro Val Thr Ala Leu Leu Leu Pro Leu Ala Leu Leu Leu
1               5                   10                  15

His Ala Ala Arg Pro Glu Val Gln Leu Leu Glu Ser Gly Gly Gly Leu
            20                  25                  30

Val Gln Pro Gly Gly Ser Leu Arg Leu Ser Cys Ala Ala Ser Gly Phe
        35                  40                  45

Thr Phe Ser Ser Tyr Gly Met His Trp Val Arg Gln Ala Pro Gly Lys
    50                  55                  60

Gly Leu Glu Trp Val Ser Ala Ile Ser Gly Ser Gly Gly Ser Thr Tyr
65                  70                  75                  80

Tyr Ala Asp Ser Val Lys Gly Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Val Ser Gly Ala Gly Arg Gly Phe Phe Asp
        115                 120                 125

Tyr Trp Gly Gln Gly Thr Leu Val Thr Val Ser Ser Gly Gly Gly Gly
    130                 135                 140

Ser Gly Gly Gly Gly Ser Gly Gly Gly Gly Ser Gln Ser Val Leu Thr
145                 150                 155                 160

Gln Pro Pro Ser Ala Ser Gly Thr Pro Gly Gln Arg Val Thr Ile Ser
                165                 170                 175

Cys Ser Gly Ser Ser Ser Asn Ile Gly Ser Asn Tyr Val Tyr Trp Tyr
            180                 185                 190

Gln Gln Leu Pro Gly Thr Ala Pro Lys Leu Leu Ile Tyr Glu Asp Asn
        195                 200                 205

Lys Arg Pro Ser Gly Val Pro Asp Arg Phe Ser Gly Ser Lys Ser Gly
    210                 215                 220

Thr Ser Ala Ser Leu Ala Ile Ser Gly Leu Arg Ser Glu Asp Glu Ala
225                 230                 235                 240

Asp Tyr Tyr Cys Ser Ser Tyr Thr Ser Ser Ser Thr Val Ile Phe Gly
                245                 250                 255

Gly Gly Thr Lys Leu Thr Val Leu Gly Gly Gly Gly Ser Gly Gly Gly
            260                 265                 270

Gly Ser Thr Thr Thr Pro Ala Pro Arg Pro Pro Thr Pro Ala Pro Thr
        275                 280                 285

Ile Ala Ser Gln Pro Leu Ser Leu Arg Pro Glu Ala Cys Arg Pro Ala
    290                 295                 300

Ala Gly Gly Ala Val His Thr Arg Gly Leu Asp Phe Ala Cys Asp Phe
305                 310                 315                 320

Trp Val Leu Val Val Val Gly Gly Val Leu Ala Cys Tyr Ser Leu Leu
                325                 330                 335

Val Thr Val Ala Phe Ile Ile Phe Trp Val Arg Ser Lys Arg Ser Arg
            340                 345                 350

Leu Leu His Ser Asp Tyr Met Asn Met Thr Pro Arg Arg Pro Gly Pro
        355                 360                 365

Thr Arg Lys His Tyr Gln Pro Tyr Ala Pro Pro Arg Asp Phe Ala Ala
    370                 375                 380

Tyr Arg Ser Arg Val Lys Phe Ser Arg Ser Ala Asp Ala Pro Ala Tyr
385                 390                 395                 400

Gln Gln Gly Gln Asn Gln Leu Tyr Asn Glu Leu Asn Leu Gly Arg Arg
                405                 410                 415

Glu Glu Tyr Asp Val Leu Asp Lys Arg Arg Gly Arg Asp Pro Glu Met
            420                 425                 430

Gly Gly Lys Pro Arg Arg Lys Asn Pro Gln Glu Gly Leu Tyr Asn Glu
        435                 440                 445

Leu Gln Lys Asp Lys Met Ala Glu Ala Tyr Ser Glu Ile Gly Met Lys
    450                 455                 460

Gly Glu Arg Arg Arg Gly Lys Gly His Asp Gly Leu Tyr Gln Gly Leu
465                 470                 475                 480

Ser Thr Ala Thr Lys Asp Thr Tyr Asp Ala Leu His Met Gln Ala Leu
                485                 490                 495

Pro Pro Arg

<210>    29
<211>    1491
<212>    DNA
<213>    Artificial Sequence

<220>
<223>    CL7 CAR

<400>    29
atggccttac cagtgaccgc cttgctcctg ccgctggcct tgctgctcca cgccgccagg      60

ccggaggtgc agctgttgga gtctggtgga ggcttggtac agcctggagg ttctcttcgc     120

ctctcctgtg cagcctccgg attcactttc agccgctacg caatgagctg ggtcagacag     180

gcaccaggta agggactgga gtgggtctct gcaattagcg gtagcggtgg tagcacttac     240

tacgcagaca gcgtgaaggg tcgcttcacc atctcacgcg acaactccaa gaacaccctg     300

tacctgcaga tgaacagcct tcgcgcagag gacactgccg tgtattactg cgcacgtagc     360

agccagggta tcttcgacat ctggggacaa ggtactctgg tcactgtctc ctcaggtgga     420

ggcggttcag gcggaggtgg atctggcggt ggcggatccc agtctgtgct gactcagcca 480

ccttcagcat ctggtactcc aggtcagcgc gtcaccatca gctgcagtgg taacaatatc 540

ggtactagac gcgtgcattg gtatcagcaa ctcccagaca ccgctcctaa gctcctgatt 600

tacagtaaga acaaccgtcc tagtggtgtg cctgatcgct tttctgggtc caagtctggc 660

acctcagcct ctctggctat cagtggactt cgctccgagg acgaggctga ctattactgc 720

gcagcatggg acgacagcct gagcggtcct gtgttcggcg gtgggaccaa actgaccgtc 780

ctaggtggcg gaggttctgg aggtggaggt tccaccacga cgccagcgcc gcgaccacca 840

acaccggcgc ccaccatcgc gtcgcagccc ctgtccctgc gcccagaggc gtgccggcca 900

gcggcggggg gcgcagtgca cacgaggggg ctggacttcg cctgtgattt ttgggtgctg 960

gtggtggttg gtggagtcct ggcttgctat agcttgctag taacagtggc ctttattatt 1020

ttctgggtga ggagtaagag gagcaggctc ctgcacagtg actacatgaa catgactccc 1080

cgccgccccg ggcccacccg caagcattac cagccctatg ccccaccacg cgacttcgca 1140

gcctatcgct ccagagtgaa gttcagcagg agcgcagacg cccccgcgta ccagcagggc 1200

cagaaccagc tctataacga gctcaatcta ggacgaagag aggagtacga tgttttggac 1260

aagagacgtg gccgggaccc tgagatgggg ggaaagccga gaaggaagaa ccctcaggaa 1320

ggcctgtaca atgaactgca gaaagataag atggcggagg cctacagtga gattgggatg 1380

aaaggcgagc gccggagggg caaggggcac gatggccttt accagggtct cagtacagcc 1440

accaaggaca cctacgacgc ccttcacatg caggccctgc cccctcgcta a 1491

<210> 30
<211> 496
<212> PRT
<213> Artificial Sequence

<220>
<223> CL7 CAR

<400> 30
Met Ala Leu Pro Val Thr Ala Leu Leu Leu Pro Leu Ala Leu Leu Leu
1               5                   10                  15

His Ala Ala Arg Pro Glu Val Gln Leu Leu Glu Ser Gly Gly Gly Leu
            20                  25                  30

Val Gln Pro Gly Gly Ser Leu Arg Leu Ser Cys Ala Ala Ser Gly Phe
        35                  40                  45

Thr Phe Ser Arg Tyr Ala Met Ser Trp Val Arg Gln Ala Pro Gly Lys
    50                  55                  60

Gly Leu Glu Trp Val Ser Ala Ile Ser Gly Ser Gly Gly Ser Thr Tyr
65                  70                  75                  80

Tyr Ala Asp Ser Val Lys Gly Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Arg Ser Ser Gln Gly Ile Phe Asp Ile Trp
        115                 120                 125

Gly Gln Gly Thr Leu Val Thr Val Ser Ser Gly Gly Gly Gly Ser Gly
    130                 135                 140

Gly Gly Gly Ser Gly Gly Gly Gly Ser Gln Ser Val Leu Thr Gln Pro
145                 150                 155                 160

Pro Ser Ala Ser Gly Thr Pro Gly Gln Arg Val Thr Ile Ser Cys Ser
                165                 170                 175

Gly Asn Asn Ile Gly Thr Arg Arg Val His Trp Tyr Gln Gln Leu Pro
            180                 185                 190

Asp Thr Ala Pro Lys Leu Leu Ile Tyr Ser Lys Asn Asn Arg Pro Ser
        195                 200                 205

Gly Val Pro Asp Arg Phe Ser Gly Ser Lys Ser Gly Thr Ser Ala Ser
    210                 215                 220

Leu Ala Ile Ser Gly Leu Arg Ser Glu Asp Glu Ala Asp Tyr Tyr Cys
225                 230                 235                 240

Ala Ala Trp Asp Asp Ser Leu Ser Gly Pro Val Phe Gly Gly Gly Thr
                245                 250                 255

Lys Leu Thr Val Leu Gly Gly Gly Gly Ser Gly Gly Gly Gly Ser Thr
            260                 265                 270

Thr Thr Pro Ala Pro Arg Pro Pro Thr Pro Ala Pro Thr Ile Ala Ser
        275                 280                 285

Gln Pro Leu Ser Leu Arg Pro Glu Ala Cys Arg Pro Ala Ala Gly Gly
    290                 295                 300

Ala Val His Thr Arg Gly Leu Asp Phe Ala Cys Asp Phe Trp Val Leu
305                 310                 315                 320

Val Val Val Gly Gly Val Leu Ala Cys Tyr Ser Leu Leu Val Thr Val
                325                 330                 335

Ala Phe Ile Ile Phe Trp Val Arg Ser Lys Arg Ser Arg Leu Leu His
            340                 345                 350

Ser Asp Tyr Met Asn Met Thr Pro Arg Arg Pro Gly Pro Thr Arg Lys
        355                 360                 365

His Tyr Gln Pro Tyr Ala Pro Pro Arg Asp Phe Ala Ala Tyr Arg Ser
    370                 375                 380

Arg Val Lys Phe Ser Arg Ser Ala Asp Ala Pro Ala Tyr Gln Gln Gly
385                 390                 395                 400

Gln Asn Gln Leu Tyr Asn Glu Leu Asn Leu Gly Arg Arg Glu Glu Tyr
                405                 410                 415

Asp Val Leu Asp Lys Arg Arg Gly Arg Asp Pro Glu Met Gly Gly Lys
            420                 425                 430

Pro Arg Arg Lys Asn Pro Gln Glu Gly Leu Tyr Asn Glu Leu Gln Lys
        435                 440                 445

Asp Lys Met Ala Glu Ala Tyr Ser Glu Ile Gly Met Lys Gly Glu Arg
    450                 455                 460

Arg Arg Gly Lys Gly His Asp Gly Leu Tyr Gln Gly Leu Ser Thr Ala
465                 470                 475                 480

Thr Lys Asp Thr Tyr Asp Ala Leu His Met Gln Ala Leu Pro Pro Arg
                485                 490                 495

<210>    31
<211>    1491
<212>    DNA
<213>    Artificial Sequence

<220>
<223>    CL10 CAR

<400>    31
atggccttac cagtgaccgc cttgctcctg ccgctggcct tgctgctcca cgccgccagg      60

ccggaggtgc agctgttgga gtctggtgga ggcttggtac agcctggagg ttctcttcgc     120

ctctcctgtg cagcctccgg attcactttc agcagctacg gtatgcattg ggtcagacag     180

gcaccaggta agggactgga gtgggtctct gcaattagcg gtagcggtgg tagcacttac     240

tacgcagaca gcgtgaaggg tcgcttcacc atctcacgcg acaactccaa gaacaccctg     300

tacctgcaga tgaacagcct tcgcgcagag gacactgccg tgtattactg cgcaagcggt     360

tacggtctga tggacgtgtg gggacaaggt actctggtca ctgtctcctc aggtggaggc     420

ggttcaggcg gaggtggatc tggcggtggc ggatcccagt ctgtgctgac tcagccacct     480

tcagcatctg gtactccagg tcagcgcgtc accatcagct gcactcgtag cagcggtatc     540

atcgcaagca actacgtgca gtggtatcag caactcccag gcaccgctcc taagctcctg     600

atttaccgca acaaccagcg ccctagtggt gtgcctgatc gcttttctgg gtccaagtct     660

ggcacctcag cctctctggc tatcagtgga cttcgctccg aggacgaggc tgactattac     720

tgcagcagct acgcaggtaa caacaacctg gtgttcggcg gtgggaccaa actgaccgtc     780

ctaggtggcg gaggttctgg aggtggaggt tccaccacga cgccagcgcc gcgaccacca     840

acaccggcgc ccaccatcgc gtcgcagccc ctgtccctgc gcccagaggc gtgccggcca     900

gcggcggggg gcgcagtgca cacgaggggg ctggacttcg cctgtgattt ttgggtgctg     960

gtggtggttg gtggagtcct ggcttgctat agcttgctag taacagtggc ctttattatt    1020

ttctgggtga ggagtaagag gagcaggctc ctgcacagtg actacatgaa catgactccc    1080

cgccgccccg ggcccacccg caagcattac cagccctatg ccccaccacg cgacttcgca 1140

gcctatcgct ccagagtgaa gttcagcagg agcgcagacg cccccgcgta ccagcagggc 1200

cagaaccagc tctataacga gctcaatcta ggacgaagag aggagtacga tgttttggac 1260

aagagacgtg gccgggaccc tgagatgggg ggaaagccga gaaggaagaa ccctcaggaa 1320

ggcctgtaca atgaactgca gaaagataag atggcggagg cctacagtga gattgggatg 1380

aaaggcgagc gccggagggg caaggggcac gatggccttt accagggtct cagtacagcc 1440

accaaggaca cctacgacgc ccttcacatg caggccctgc cccctcgcta a 1491

<210> 32
<211> 496
<212> PRT
<213> Artificial Sequence

<220>
<223> CL10 CAR

<400> 32
Met Ala Leu Pro Val Thr Ala Leu Leu Leu Pro Leu Ala Leu Leu Leu
1               5                   10                  15

His Ala Ala Arg Pro Glu Val Gln Leu Leu Glu Ser Gly Gly Gly Leu
            20                  25                  30

Val Gln Pro Gly Gly Ser Leu Arg Leu Ser Cys Ala Ala Ser Gly Phe
        35                  40                  45

Thr Phe Ser Ser Tyr Gly Met His Trp Val Arg Gln Ala Pro Gly Lys
    50                  55                  60

Gly Leu Glu Trp Val Ser Ala Ile Ser Gly Ser Gly Gly Ser Thr Tyr
65                  70                  75                  80

Tyr Ala Asp Ser Val Lys Gly Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Ser Gly Tyr Gly Leu Met Asp Val Trp Gly
        115                 120                 125

Gln Gly Thr Leu Val Thr Val Ser Ser Gly Gly Gly Gly Ser Gly Gly
    130                 135                 140

Gly Gly Ser Gly Gly Gly Gly Ser Gln Ser Val Leu Thr Gln Pro Pro
145                 150                 155                 160

Ser Ala Ser Gly Thr Pro Gly Gln Arg Val Thr Ile Ser Cys Thr Arg
                165                 170                 175

Ser Ser Gly Ile Ile Ala Ser Asn Tyr Val Gln Trp Tyr Gln Gln Leu
            180                 185                 190

Pro Gly Thr Ala Pro Lys Leu Leu Ile Tyr Arg Asn Asn Gln Arg Pro
        195                 200                 205

Ser Gly Val Pro Asp Arg Phe Ser Gly Ser Lys Ser Gly Thr Ser Ala
    210                 215                 220

Ser Leu Ala Ile Ser Gly Leu Arg Ser Glu Asp Glu Ala Asp Tyr Tyr
225                 230                 235                 240

Cys Ser Ser Tyr Ala Gly Asn Asn Asn Leu Val Phe Gly Gly Gly Thr
                245                 250                 255

Lys Leu Thr Val Leu Gly Gly Gly Gly Ser Gly Gly Gly Gly Ser Thr
            260                 265                 270

Thr Thr Pro Ala Pro Arg Pro Pro Thr Pro Ala Pro Thr Ile Ala Ser
        275                 280                 285

Gln Pro Leu Ser Leu Arg Pro Glu Ala Cys Arg Pro Ala Ala Gly Gly
    290                 295                 300

Ala Val His Thr Arg Gly Leu Asp Phe Ala Cys Asp Phe Trp Val Leu
305                 310                 315                 320

Val Val Val Gly Gly Val Leu Ala Cys Tyr Ser Leu Leu Val Thr Val
                325                 330                 335

Ala Phe Ile Ile Phe Trp Val Arg Ser Lys Arg Ser Arg Leu Leu His
            340                 345                 350

Ser Asp Tyr Met Asn Met Thr Pro Arg Arg Pro Gly Pro Thr Arg Lys
        355                 360                 365

His Tyr Gln Pro Tyr Ala Pro Pro Arg Asp Phe Ala Ala Tyr Arg Ser
    370                 375                 380

Arg Val Lys Phe Ser Arg Ser Ala Asp Ala Pro Ala Tyr Gln Gln Gly
385                 390                 395                 400

Gln Asn Gln Leu Tyr Asn Glu Leu Asn Leu Gly Arg Arg Glu Glu Tyr
                405                 410                 415

Asp Val Leu Asp Lys Arg Arg Gly Arg Asp Pro Glu Met Gly Gly Lys
            420                 425                 430

Pro Arg Arg Lys Asn Pro Gln Glu Gly Leu Tyr Asn Glu Leu Gln Lys
        435                 440                 445

Asp Lys Met Ala Glu Ala Tyr Ser Glu Ile Gly Met Lys Gly Glu Arg
    450                 455                 460

Arg Arg Gly Lys Gly His Asp Gly Leu Tyr Gln Gly Leu Ser Thr Ala
465                 470                 475                 480

Thr Lys Asp Thr Tyr Asp Ala Leu His Met Gln Ala Leu Pro Pro Arg
                485                 490                 495


<210>    33
<211>    1488
<212>    DNA
<213>    Artificial Sequence

<220>

<223>    SL18 CAR

<400>    33
atggccttac cagtgaccgc cttgctcctg ccgctggcct tgctgctcca cgccgccagg      60

ccgcgagtgc agctgctgga aagtggaggt ggactggtgc agcctggcgg cagcctgcgc     120

ctgagctgtg ccgccagcgg attcaccttc agcgattatc atatgcattg ggttcgccaa     180

gcacctggca aaggcctgga atgggtgagc accatcagca gcagcggcgg ctatacctat     240

tatgccgaaa gcgtgaaaag ccgctttacc atcagccgcg ataacagcaa aaacaccctg     300

tatctgcaga tgaacagcct gcgcgccgag gacaccgcag tctactactg tgcccgatcg     360

atacgcctgc ctctggatta ttggggacaa ggtactctgg tgaccgtgag cagcagtgga     420

ggaggtagcg gaggtggtgg atctggaggt ggaggtagtc agagcgtgct gacccagcct     480

cctagcgcct ccggtacacc aggacagcgc gtgactatta gctgtagcgg caacaacatc     540

ggcagcaaag gcgtgcattg gtatcagcaa ctgcctggaa ctgcacctaa gctgctgatc     600

tatgaagata gcaaacgccc tagcggcgtg cgtgatcgct ttagcggtag caaatcaggc     660

accagcgcca gcctggccat cagcggcctt cgctccgaag atgaagccga ttattattgt     720

cagagctatg atagcaccaa aggcgtggtg tttggtggcg gtaccaagct gaccgtgctg     780

ggtggcggag gttctggagg tggaggttcc accacgacgc cagcgccgcg accaccaaca     840

ccggcgccca ccatcgcgtc gcagcccctg tccctgcgcc cagaggcgtg ccggccagcg     900

gcggggggcg cagtgcacac gagggggctg gacttcgcct gtgatttttg ggtgctggtg     960

gtggttggtg gagtcctggc ttgctatagc ttgctagtaa cagtggcctt tattattttc    1020

tgggtgagga gtaagaggag caggctcctg cacagtgact acatgaacat gactccccgc    1080

cgccccgggc ccacccgcaa gcattaccag ccctatgccc caccacgcga cttcgcagcc    1140

tatcgctcca gagtgaagtt cagcaggagc gcagacgccc ccgcgtacca gcagggccag    1200

aaccagctct ataacgagct caatctagga cgaagagagg agtacgatgt tttggacaag    1260

agacgtggcc gggaccctga gatgggggga aagccgagaa ggaagaaccc tcaggaaggc    1320

ctgtacaatg aactgcagaa agataagatg gcggaggcct acagtgagat tgggatgaaa    1380

ggcgagcgcc ggaggggcaa ggggcacgat ggcctttacc agggtctcag tacagccacc    1440

aaggacacct acgacgccct tcacatgcag gccctgcccc ctcgctaa                 1488

<210>    34
<211>    495
<212>    PRT
<213>    Artificial Sequence

<220>
<223>    SL18 CAR

<400>    34
Met Ala Leu Pro Val Thr Ala Leu Leu Leu Pro Leu Ala Leu Leu Leu
1               5                   10                  15

His Ala Ala Arg Pro Arg Val Gln Leu Leu Glu Ser Gly Gly Gly Leu
            20                  25                  30

Val Gln Pro Gly Gly Ser Leu Arg Leu Ser Cys Ala Ala Ser Gly Phe
        35                  40                  45

Thr Phe Ser Asp Tyr His Met His Trp Val Arg Gln Ala Pro Gly Lys
    50                  55                  60

Gly Leu Glu Trp Val Ser Thr Ile Ser Ser Ser Gly Gly Tyr Thr Tyr
65                  70                  75                  80

Tyr Ala Glu Ser Val Lys Ser Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Arg Ser Ile Arg Leu Pro Leu Asp Tyr Trp
        115                 120                 125

Gly Gln Gly Thr Leu Val Thr Val Ser Ser Ser Gly Gly Gly Ser Gly
    130                 135                 140

Gly Gly Gly Ser Gly Gly Gly Gly Ser Gln Ser Val Leu Thr Gln Pro
145                 150                 155                 160

Pro Ser Ala Ser Gly Thr Pro Gly Gln Arg Val Thr Ile Ser Cys Ser
                165                 170                 175

Gly Asn Asn Ile Gly Ser Lys Gly Val His Trp Tyr Gln Gln Leu Pro
            180                 185                 190

Gly Thr Ala Pro Lys Leu Leu Ile Tyr Glu Asp Ser Lys Arg Pro Ser
        195                 200                 205

Gly Val Arg Asp Arg Phe Ser Gly Ser Lys Ser Gly Thr Ser Ala Ser
    210                 215                 220

Leu Ala Ile Ser Gly Leu Arg Ser Glu Asp Glu Ala Asp Tyr Tyr Cys
225                 230                 235                 240

Gln Ser Tyr Asp Ser Thr Lys Gly Val Val Phe Gly Gly Gly Thr Lys
                245                 250                 255

Leu Thr Val Leu Gly Gly Gly Gly Ser Gly Gly Gly Gly Ser Thr Thr
            260                 265                 270

Thr Pro Ala Pro Arg Pro Pro Thr Pro Ala Pro Thr Ile Ala Ser Gln
        275                 280                 285

Pro Leu Ser Leu Arg Pro Glu Ala Cys Arg Pro Ala Ala Gly Gly Ala
    290                 295                 300

Val His Thr Arg Gly Leu Asp Phe Ala Cys Asp Phe Trp Val Leu Val
305                 310                 315                 320

Val Val Gly Gly Val Leu Ala Cys Tyr Ser Leu Leu Val Thr Val Ala

                        325                         330                         335

Phe Ile Ile Phe Trp Val Arg Ser Lys Arg Ser Arg Leu Leu His Ser
                340                         345                         350

Asp Tyr Met Asn Met Thr Pro Arg Arg Pro Gly Pro Thr Arg Lys His
            355                         360                         365

Tyr Gln Pro Tyr Ala Pro Pro Arg Asp Phe Ala Ala Tyr Arg Ser Arg
    370                         375                         380

Val Lys Phe Ser Arg Ser Ala Asp Ala Pro Ala Tyr Gln Gln Gly Gln
385                         390                         395                         400

Asn Gln Leu Tyr Asn Glu Leu Asn Leu Gly Arg Arg Glu Glu Tyr Asp
                        405                         410                         415

Val Leu Asp Lys Arg Arg Gly Arg Asp Pro Glu Met Gly Gly Lys Pro
                420                         425                         430

Arg Arg Lys Asn Pro Gln Glu Gly Leu Tyr Asn Glu Leu Gln Lys Asp
            435                         440                         445

Lys Met Ala Glu Ala Tyr Ser Glu Ile Gly Met Lys Gly Glu Arg Arg
    450                         455                         460

Arg Gly Lys Gly His Asp Gly Leu Tyr Gln Gly Leu Ser Thr Ala Thr
465                         470                         475                         480

Lys Asp Thr Tyr Asp Ala Leu His Met Gln Ala Leu Pro Pro Arg
                        485                         490                         495


<210>    35
<211>    1260
<212>    DNA
<213>    Artificial Sequence

<220>
<223>    CD300c ECD CAR


<400>    35
atggccttac cagtgaccgc cttgctcctg ccgctggcct tgctgctcca cgccgccagg          60

ccgggctatt ttcctctgag ccaccccatg accgtggcgg gccccgtggg gggatccctg         120

agtgtgcagt gtcgctatga gaaggaacac aggaccctca acaaattctg gtgcagacca         180

ccacagattc tccgatgtga caagattgtg gagaccaaag ggtcagcagg gaaaaggaat         240

ggccgagtgt ccatcaggga cagtcctgca aacctcagct tcacagtgac cctggagaat         300

ctcacagagg aggacgcagg cacctactgg tgtggggtgg atacaccgtg gctccgagac         360

tttcatgatc ccattgtcga ggttgaggtg tccgtgttcc cggccgggac gaccacagcc         420

tccagccccc agagctccat gggcacctca ggtcctccca cgaagctgcc cgtgcacacc         480

tggcccagcg tgaccagaaa ggacagcccc gaacccagcc cacaccctgg ctccctgttc         540

agcaatgtcc gcggtggcgg aggttctgga ggtggaggtt ccaccacgac gccagcgccg         600

cgaccaccaa caccggcgcc caccatcgcg tcgcagcccc tgtccctgcg cccagaggcg 660

tgccggccag cggcgggggg cgcagtgcac acgagggggc tggacttcgc ctgtgatttt 720

tgggtgctgg tggtggttgg tggagtcctg gcttgctata gcttgctagt aacagtggcc 780

tttattattt tctgggtgag gagtaagagg agcaggctcc tgcacagtga ctacatgaac 840

atgactcccc gccgccccgg gcccacccgc aagcattacc agccctatgc cccaccacgc 900

gacttcgcag cctatcgctc cagagtgaag ttcagcagga gcgcagacgc ccccgcgtac 960

cagcagggcc agaaccagct ctataacgag ctcaatctag gacgaagaga ggagtacgat 1020

gttttggaca agagacgtgg ccgggaccct gagatggggg gaaagccgag aaggaagaac 1080

cctcaggaag gcctgtacaa tgaactgcag aaagataaga tggcggaggc ctacagtgag 1140

attgggatga aaggcgagcg ccggaggggc aaggggcacg atggccttta ccagggtctc 1200

agtacagcca ccaaggacac ctacgacgcc cttcacatgc aggccctgcc ccctcgctaa 1260

1260

<210> 36
<211> 419
<212> PRT
<213> Artificial Sequence

<220>
<223> CD300c ECD CAR

<400> 36
Met Ala Leu Pro Val Thr Ala Leu Leu Leu Pro Leu Ala Leu Leu Leu
1               5                   10                  15

His Ala Ala Arg Pro Gly Tyr Phe Pro Leu Ser His Pro Met Thr Val
            20                  25                  30

Ala Gly Pro Val Gly Gly Ser Leu Ser Val Gln Cys Arg Tyr Glu Lys
        35                  40                  45

Glu His Arg Thr Leu Asn Lys Phe Trp Cys Arg Pro Pro Gln Ile Leu
    50                  55                  60

Arg Cys Asp Lys Ile Val Glu Thr Lys Gly Ser Ala Gly Lys Arg Asn
65                  70                  75                  80

Gly Arg Val Ser Ile Arg Asp Ser Pro Ala Asn Leu Ser Phe Thr Val
                85                  90                  95

Thr Leu Glu Asn Leu Thr Glu Glu Asp Ala Gly Thr Tyr Trp Cys Gly
            100                 105                 110

Val Asp Thr Pro Trp Leu Arg Asp Phe His Asp Pro Ile Val Glu Val
        115                 120                 125

Glu Val Ser Val Phe Pro Ala Gly Thr Thr Thr Ala Ser Ser Pro Gln
    130                 135                 140

Ser Ser Met Gly Thr Ser Gly Pro Pro Thr Lys Leu Pro Val His Thr

145                 150                 155                 160

Trp Pro Ser Val Thr Arg Lys Asp Ser Pro Glu Pro Ser Pro His Pro
                165                 170                 175

Gly Ser Leu Phe Ser Asn Val Arg Gly Gly Gly Gly Ser Gly Gly Gly
            180                 185                 190

Gly Ser Thr Thr Thr Pro Ala Pro Arg Pro Pro Thr Pro Ala Pro Thr
        195                 200                 205

Ile Ala Ser Gln Pro Leu Ser Leu Arg Pro Glu Ala Cys Arg Pro Ala
    210                 215                 220

Ala Gly Gly Ala Val His Thr Arg Gly Leu Asp Phe Ala Cys Asp Phe
225                 230                 235                 240

Trp Val Leu Val Val Val Gly Gly Val Leu Ala Cys Tyr Ser Leu Leu
                245                 250                 255

Val Thr Val Ala Phe Ile Ile Phe Trp Val Arg Ser Lys Arg Ser Arg
            260                 265                 270

Leu Leu His Ser Asp Tyr Met Asn Met Thr Pro Arg Arg Pro Gly Pro
        275                 280                 285

Thr Arg Lys His Tyr Gln Pro Tyr Ala Pro Pro Arg Asp Phe Ala Ala
    290                 295                 300

Tyr Arg Ser Arg Val Lys Phe Ser Arg Ser Ala Asp Ala Pro Ala Tyr
305                 310                 315                 320

Gln Gln Gly Gln Asn Gln Leu Tyr Asn Glu Leu Asn Leu Gly Arg Arg
                325                 330                 335

Glu Glu Tyr Asp Val Leu Asp Lys Arg Arg Gly Arg Asp Pro Glu Met
            340                 345                 350

Gly Gly Lys Pro Arg Arg Lys Asn Pro Gln Glu Gly Leu Tyr Asn Glu
        355                 360                 365

Leu Gln Lys Asp Lys Met Ala Glu Ala Tyr Ser Glu Ile Gly Met Lys
    370                 375                 380

Gly Glu Arg Arg Arg Gly Lys Gly His Asp Gly Leu Tyr Gln Gly Leu
385                 390                 395                 400

Ser Thr Ala Thr Lys Asp Thr Tyr Asp Ala Leu His Met Gln Ala Leu
                405                 410                 415

Pro Pro Arg

## Description

### Technical Field

The present disclosure relates to a chimeric antigen receptor that specifically binds to a CD300c antigen or a receptor thereof, immune cells expressing the same, uses thereof, and the like.

### Background Art

Cancer is one of the diseases that account for the largest share of the causes of death in modern people. This disease is caused by changes in normal cells due to genetic mutations that result from various causes and refers to a malignant tumor that does not follow differentiation, proliferation, growth pattern, or the like of normal cells. Cancer is characterized by "uncontrolled cell growth." This abnormal cell growth causes formation of a mass of cells called a tumor, which infiltrates the surrounding tissues and, in severe cases, may metastasize to other organs of the body. Cancer is an intractable chronic disease that is not fundamentally cured in many cases even if it is treated with surgery, radiotherapy, chemotherapy, and the like, causes pain to patients, and ultimately leads to death. In particular, in recent years, the global cancer incidence rate is increasing by 5% or higher every year due to increased elderly population, environmental deterioration, or the like. According to the WHO report, it is estimated that within the next 25 years the number of cancer patients will increase to 30 million, of which 20 million will die from cancer.

Cancer drug treatments, that is, cancer chemotherapies are generally cytotoxic compounds, and treat cancer by attacking and killing cancer cells. However, these chemotherapies exhibit high adverse effects since they damage not only cancer cells but also normal cells. Thus, targeted cancer chemotherapies have been developed to decrease adverse effects. These targeted cancer chemotherapies were able to exhibit decreased adverse effects, but had a limitation in that resistance occurs with a high probability. Therefore, in recent years, interest in cancer immunotherapies, which use the body's immune system to decrease problems due to toxicity and resistance, is rapidly increasing. As an example of such cancer immunotherapies, immune checkpoint inhibitors have been developed which specifically bind to PD-L1 on the surface of cancer cells and inhibit its binding to PD-1 on T cells so that T cells are activated and attack cancer cells. However, even these immune checkpoint inhibitors are not effective in various types of cancer. Therefore, there is a need to develop novel cancer immune therapeutics that exhibit an equivalent therapeutic effect in various cancers (Korean Patent Laid-Open Publication No. 10-2016-0016725).

Meanwhile, chimeric antigen receptors (CARs) are artificial receptors designed to deliver antigen specificity to T cells and comprise an antigen-specific domain that activates T cells and provides specific immunity, a transmembrane domain, an intracellular domain, and the like. Recently, studies are actively conducted on cancer immunotherapy using cells into which a gene encoding such a chimeric antigen receptor has been introduced, that is, a method for treating cancer through a therapy in which T cells are collected from a patient, a gene encoding a chimeric antigen receptor is introduced into these T cells and amplified, and transferred back into the patient.

### Disclosure of Invention

### Technical Problem

The present disclosure has been made to solve the problems of the prior art as described above. An object of the present disclosure is to provide a chimeric antigen receptor that specifically binds to a CD300c antigen or a receptor thereof, immune cells expressing the same, a composition for preventing or treating cancer using the same, and the like.

However, the technical problem to be solved by the present disclosure is not limited to the above-mentioned problems, and other problems which are not mentioned will be clearly understood by those skilled in the art from the following description.

### Solution to Problem

According to the present disclosure, there is provided a chimeric antigen receptor that specifically binds to a CD300c antigen or a receptor thereof.

In an embodiment of the present disclosure, the chimeric antigen receptor may preferably comprise an antibody, a single domain antibody, a single chain variable fragment (scFv), or the like, which specifically binds to a CD300c antigen, or comprise a CD300c antigen, thereby specifically recognizing and binding to the CD300c antigen or a receptor thereof. The CD300c antigen may comprise the entire CD300c antigen sequence or only an extracellular domain (ECD) of the CD300c antigen sequence, for binding to a receptor thereof. The extracellular domain sequence of the CD300c antigen may comprise the amino acid sequence represented by SEQ ID NO: 14. The extracellular domain sequence may comprise an amino acid sequence having preferably 90% or higher, more preferably 95% or higher, and most preferably 98% or higher sequence homology to SEQ ID NO: 14. In addition, the antibody, the single domain antibody, the single chain variable fragment, or the like may comprise the amino acid sequence represented by SEQ ID NO: 4, 6, 8, 10, or 12 for binding to the CD300c antigen. The antibody, the single domain antibody, the single chain variable fragment, or the like may comprise an amino acid sequence having preferably 90% or higher, more preferably 95% or higher, and most preferably 98% or higher sequence homology to SEQ ID NO: 4, 6, 8, 10, or 12. The "% sequence homology" with respect to amino acid sequences is determined by comparing two optimally aligned sequences over a comparison window, wherein the portion of the amino acid sequence in the comparison window may include additions or deletions (that is, gaps) as compared to the reference sequence (that does not include additions or deletions) for optimal alignment of the two sequences. However, any amino acid sequence may be used as long as it is a sequence capable of specifically binding to the CD300c antigen or a receptor thereof.

In another embodiment of the present disclosure, the chimeric antigen receptor may preferably further comprise a signal peptide, a GS linker, a transmembrane domain, an intracytoplasmic domain, and the like, and any component may be further included therein as long as it is a component of a commonly known chimeric antigen receptor.

In yet another embodiment of the present disclosure, preferably, the signal peptide may comprise a CD8α signal peptide sequence; the transmembrane domain may comprise sequences such as a CD8 hinge (hinge of cluster of differentiation 8) and a CD28 transmembrane domain; and the intracytoplasmic domain may comprise sequences such as a CD28 intracellular domain and a CD3ζ intracellular domain.

In still yet another embodiment of the present disclosure, the chimeric antigen receptor specifically recognizes and/or binds to the CD300c antigen or a receptor thereof, and thus can be used for the treatment of cancer that expresses the CD300c antigen on its surface.

In addition, according to the present disclosure, there is provided a recombinant vector expressing the chimeric antigen receptor.

In addition, according to the present disclosure, there is provided an immune cell transformed with the recombinant vector.

In an embodiment of the present disclosure, the immune cells preferably include monocytes, macrophages, T cells, natural killer cells (NK cells), dendritic cells, and the like, and any immune cells may be included therein as long as they can be used for the treatment of cancer.

In addition, according to the present disclosure, there is provided a pharmaceutical composition for preventing or treating cancer that expresses a CD300c antigen or a CD300c receptor, the pharmaceutical composition comprising the immune cells as an active ingredient.

In an embodiment of the present disclosure, the cancer may be colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, stomach cancer, bone cancer, blood cancer, or the like. However, the cancer is not limited thereto and may include any type of cancer in which the CD300c antigen or the CD300c receptor is expressed on the surface.

In another embodiment of the present disclosure, the pharmaceutical composition may further comprise (an)other conventional cancer chemotherapy(ies). The chemotherapy may preferably be, but is not limited to, doxorubicin, cisplatin, gemcitabine, oxaliplatin, 5-FU, cetuximab, panitumumab, nimotuzumab, necitumumab, cancer antigen, anticancer virus, or the like, and may include any substance as long as it is currently used as a chemotherapy. The cancer antigen is a cancer vaccine specific to carcinoma and may preferably be NY-ESO-1 as a bladder cancer-specific cancer antigen, HER2 as a breast cancer-specific cancer antigen, CEA as a colorectal cancer-specific cancer antigen, and VEGFR1 or VEGFR2 as a lung cancer-specific cancer antigen. However, the cancer antigen is not limited thereto and may include any type of cancer antigen as long as it is known as a cancer vaccine. Examples of the anticancer virus include Imlygic and Pexa-Vec. However, the anticancer virus is not limited thereto and may include any anticancer virus as long as it is known as an anticancer virus. In a case where the cancer therapy is further included, such a therapy may preferably be co-administered with the immune cells of the present disclosure, may be in a form of being included in the immune cells, may be in a form of being bound to the chimeric antigen receptor that specifically binds to a CD300c antigen or a receptor thereof, or may be included together in a vehicle therefor. However, the present disclosure is not limited thereto.

In yet another embodiment of the present disclosure, the pharmaceutical composition is characterized in that it inhibits proliferation, survival, metastasis, recurrence, therapy resistance, or the like of cancer or cancer stem cells. However, the effect is not limited thereto and may include any effect exerted by the pharmaceutical composition of the present disclosure.

In addition, according to the present disclosure, there is provided a method for treating cancer, comprising a step of administering to an individual a composition that comprises immune cells as an active ingredient, wherein the immune cells express a chimeric antigen receptor that specifically binds to a CD300c antigen or a receptor thereof.

In addition, according to the present disclosure, there is provided a use of a composition that comprises immune cells as an active ingredient, for preventing or treating cancer, wherein the immune cells express a chimeric antigen receptor that specifically binds to a CD300c antigen or a receptor thereof.

In addition, according to the present disclosure, there is provided a use of immune cells, which express a chimeric antigen receptor that specifically binds to a CD300c antigen or a receptor thereof, for the manufacture of a medicament for use in cancer treatment.

### Advantageous Effects of Invention

The chimeric antigen receptor that specifically binds to a CD300c antigen or a receptor thereof according to the present disclosure specifically binds, with high binding affinity, to the CD300c antigen or a receptor thereof expressed on the surface of various cancers, which activates T cells and at the same time inhibits proliferation of cancer cells. Thus, the chimeric antigen receptor can be effectively used as an immunotherapeutic agent for various cancers. In addition, it was identified that use of immune cells, which express a chimeric antigen receptor that specifically binds to a CD300c antigen or a receptor thereof, of the present disclosure allows a therapeutic effect caused by the immune cells themselves as well as inhibited production of the CD300c protein caused by the chimeric antigen receptor, which leads to activation of T cells and inhibited proliferation of cancer cells, so that growth, development, metastasis, and the like of cancer cells can be effectively suppressed. In addition, it is expected that in a case where resistant cancer cells showing the ability to resist apoptosis are treated with immune cells, which express a chimeric antigen receptor that specifically binds to a CD300c antigen or a receptor thereof, according to the present disclosure such treatment can remarkably weaken these capacities of cancer cells, thereby showing excellent efficacy in preventing cancer recurrence.

### Brief Description of Drawings

FIG. 1 schematically illustrates gene arrangement for constructing a chimeric antigen receptor that specifically binds to a CD300c antigen or a receptor thereof according to an embodiment of the present disclosure.
FIG. 2 illustrates a vector map for constructing a chimeric antigen receptor that specifically binds to a CD300c antigen or a receptor thereof according to an embodiment of the present disclosure.
FIG. 3 illustrates results obtained by identifying, through Western blotting, the Jurkat cell line that expresses a chimeric antigen receptor that specifically binds to a CD300c antigen or a receptor thereof according to an embodiment of the present disclosure.
FIG. 4 illustrates results obtained by identifying anticancer effects of the Jurkat cell line expressing the chimeric antigen receptor that specifically binds to the CD300c antigen or a receptor thereof according to an embodiment of the present disclosure.

### Best Mode for Carrying out Invention

The immune cells expressing the chimeric antigen receptor that specifically binds to a CD300c antigen or a receptor thereof of the present disclosure specifically bind to the CD300c antigen or a receptor thereof on the surface of cancer cells and effectively inhibit the mechanism of CD300c, so that growth of cancer cells and development of cancer can be effectively inhibited. Thus, such immune cells can be effectively used for the treatment of various cancers that express the CD300c antigen or a receptor thereof on the surface.

As used herein, the term "antibody" refers to an immunoglobulin molecule that is immunologically reactive with a specific antigen, and includes all of polyclonal antibodies, monoclonal antibodies, and functional fragments thereof. In addition, the term may include forms produced by genetic engineering, such as chimeric antibodies (for example, humanized murine antibodies) and heterologous antibodies (for example, bispecific antibodies). Among these, the monoclonal antibodies are antibodies that exhibit single binding specificity and affinity against a single antigenic site (epitope). Unlike polyclonal antibodies including antibodies that exhibit specificity against different epitopes, the monoclonal antibodies exhibit binding specificity and affinity against a single epitope on an antigen, which allows for easy quality control as a therapeutic agent. In particular, the anti-CD300c monoclonal antibody of the present disclosure not only exhibits anticancer activity by itself by specifically binding to CD300c-expressing cancer cells, but also stimulates immune cells, thereby exhibiting maximized cancer cell-dependent anticancer activity. The antibody includes variable region(s) of a heavy chain and/or a light chain in terms of the constitution, wherein the variable region includes, as a primary structure thereof, a portion that forms an antigen-binding site of the antibody molecule. The antibody of the present disclosure may be composed of a partial fragment containing the variable region. Preferably, the variable region may be replaced by a soluble receptor for CD300c. However, the variable region is not limited thereto and may include anything as long as the thus formed antibody exhibits the same effect as the anti-CD300c monoclonal antibody of the present disclosure.

As used herein, the "single domain antibody" is an antibody specific for the CD300c antigen, in which a CDR is a portion of a single domain polypeptide, and may be generally produced using only two heavy chains and an antigen-binding site. However, the single domain antibody may include all of heavy chain antibodies, antibodies naturally devoid of light chains, single-domain antibodies derived from conventional 4-chain antibodies, engineered antibodies, and single domain scaffolds other than those derived from antibodies.

As used herein, the term "single-chain variable fragment (scFv)" refers to a protein in which light chain and heavy chain variable regions of an antibody are linked to each other via a linker consisting of a peptide sequence having about 15 amino acid residues. The scFv may be in an order of light chain variable domain-linker-heavy chain variable region, or an order of heavy chain variable region-linker-light chain variable region, and has the same or similar antigen specificity as its original antibody. The linking site is a hydrophilic flexible peptide chain mainly composed of glycine and serine. The 15-amino acid sequence of "(Gly-Gly-Gly-Gly-Ser)₃ or a sequence similar thereto is mainly used. The antibody refers to an immunoglobulin molecule that is immunologically reactive with a specific antigen, and includes all of polyclonal antibodies, monoclonal antibodies, and functional fragments thereof. In addition, the term may include forms produced by genetic engineering, such as chimeric antibodies (for example, humanized murine antibodies) and heterologous antibodies (for example, bispecific antibodies). Among these, the monoclonal antibodies are antibodies that exhibit single binding specificity and affinity against a single antigenic site (epitope). Unlike polyclonal antibodies including antibodies that exhibit specificity against different epitopes, the monoclonal antibodies exhibit binding specificity and affinity against a single epitope on an antigen, which allows for easy quality control as a therapeutic agent. In particular, the anti-CD300c monoclonal antibody of the present disclosure not only exhibits anticancer activity by itself by specifically binding to CD300c-expressing cancer cells, but also stimulates immune cells, thereby exhibiting maximized cancer cell-dependent anticancer activity. The antibody includes variable region(s) of a heavy chain and/or a light chain in terms of the constitution, wherein the variable region includes, as a primary structure thereof, a portion that forms an antigen-binding site of the antibody molecule. The antibody of the present disclosure may be composed of a partial fragment containing the variable region.

As used herein, the term "prevention" means any action that inhibits or delays onset of diseases such as cancer by administration of the pharmaceutical composition according to the present disclosure.

As used herein, the term "treatment" means any action that ameliorates or beneficially alters symptoms of cancer or the like by administration of the pharmaceutical composition according to the present disclosure.

As used herein, the term "individual" refers to a subject to which the pharmaceutical composition of the present disclosure can be administered, and the subject is not limited.

As used herein, the term "pharmaceutical composition" may be characterized by being in the form of capsules, tablets, granules, injections, ointments, powders, or beverages, and the pharmaceutical composition may be characterized by being for application to humans. The pharmaceutical composition may be formulated in the form of oral preparations such as powders, granules, capsules, tablets, and aqueous suspensions, preparations for external use, suppositories, and sterile injectable solutions, respectively, according to conventional methods, and used. However, the pharmaceutical composition is not limited thereto. The pharmaceutical composition of the present disclosure may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The preparations of the pharmaceutical composition of the present disclosure may be prepared in various ways by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, as examples of carriers, diluents, or excipients suitable for making preparations, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, mineral oil, or the like may be used. In addition, a filler, an anti-coagulant, a lubricant, a wetting agent, a fragrance, an emulsifier, a preservative, and the like may further be included.

The route of administration of the pharmaceutical composition according to the present disclosure includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred. As used herein, the term "parenteral" includes subcutaneous, intradermal, intravenous, intramuscular, intraarticular, intrabursal, intrasternal, intradural, intralesional, and intracranial injection or infusion techniques. The pharmaceutical composition of the present disclosure may also be administered in the form of suppositories for rectal administration.

The pharmaceutical composition of the present disclosure may vary widely depending on a variety of factors, including activity of a certain compound used, the patient's age, body weight, general health status, sex, diet, time of administration, route of administration, rate of excretion, drug combination, and severity of a certain disease to be prevented or treated. A dose of the pharmaceutical composition may vary depending on the patient's condition, body weight, severity of disease, drug form, route of administration, and duration, and may be appropriately selected by those skilled in the art. The pharmaceutical composition may be administered in an amount of 0.0001 to 500 mg/kg or 0.001 to 500 mg/kg, per day. Administration may be made once a day or several times a day. The dose is not intended to limit the scope of the invention in any way. The pharmaceutical composition according to the present disclosure may be formulated in the form of pills, sugar-coated tablets, capsules, liquids, gels, syrups, slurries, or suspensions.

Hereinafter, the following examples are provided to help the understanding of the present disclosure. However, the following examples are only provided for easier understanding of the present disclosure, and the scope of the present disclosure is not limited by the following examples.

### Examples

### Example 1: Construction of expression vector for chimeric antigen receptor that specifically binds to CD300c antigen or receptor thereof

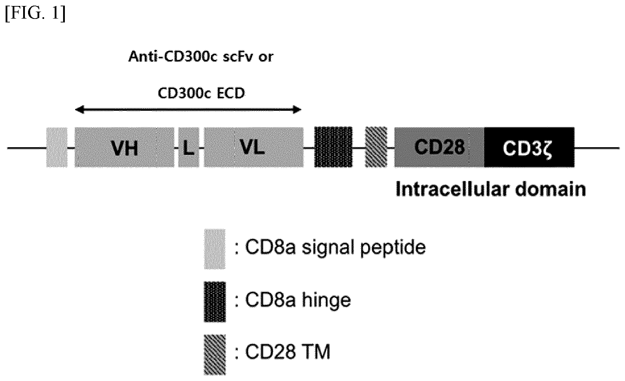

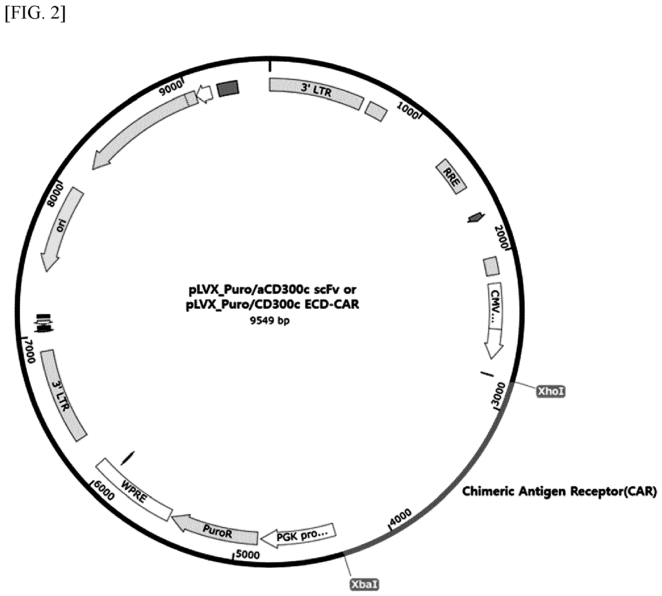

To produce a chimeric antigen receptor that specifically binds to a CD300c antigen or a receptor thereof, the following sequences were sequentially inserted into the pLVX-Puro vector (Addgene) to construct an expression vector for the chimeric antigen receptor (pLVX-Puro/aCD300c scFv or pLVX-Puro/CD300c ECD-CAR) that specifically binds to the CD300c antigen or a receptor thereof: a CD8α signal peptide (whose DNA sequence is represented by SEQ ID NO: 1) comprising the amino acid sequence represented by SEQ ID NO: 2 which allows the synthesized protein to pass through the cell membrane and move to the correct position; each of CK1, CL6, CL7, CL10, and SL18 comprising the amino acid sequences represented by SEQ ID NOs: 4, 6, 8, 10, and 12, respectively, which specifically bind to a CD300c antigen or a receptor thereof and are anti-CD300c single chain variable fragments (aCD300c scFvs; whose DNA sequences are represented by SEQ ID NO: 3, 5, 7, 9, and 11, respectively), or a CD300c extracellular domain (ECD) antigen comprising the amino acid sequence represented by SEQ ID NO: 14 (whose DNA sequence is represented by SEQ ID NO: 13); a GS linker comprising the amino acid sequence represented by SEQ ID NO: 16 (whose DNA sequence is represented by SEQ ID NO: 15); a CD8 hinge comprising the amino acid sequence represented by SEQ ID NO: 18 (whose DNA sequence is represented by SEQ ID NO: 17) and a CD28 transmembrane domain comprising the amino acid sequence represented by SEQ ID NO: 20 (whose DNA sequence is represented by SEQ ID NO: 19) as a transmembrane domain; a CD28 intracellular domain comprising the amino acid sequence represented by SEQ ID NO: 22 (whose DNA sequence is represented by SEQ ID NO: 21) as an intracytoplasmic domain involved in signal transduction for macrophage activation; and a CD3ζ intracellular domain comprising the amino acid sequence represented by SEQ ID NO: 24 (whose DNA sequence is represented by SEQ ID NO: 23). The gene and protein sequence combinations for the prepared respective vectors are shown in Table 1. A schematic diagram of the gene arrangement is illustrated in FIG. 1, and an example of the constructed vector map is illustrated in FIG. 2.

**[Table 1]**

| CAR name | DNA sequence combination | Protein sequence combination | SEQ ID NOs |
|---|---|---|---|
| CK1 CAR | Combination of SEQ ID NOs: 1,3, 15, 17, 19,21,23 | Combination of SEQ ID NOs: 2, 4, 16, 18, 20, 22, 24 | 25 and 26 |
| CL6 CAR | Combination of SEQ ID NOs: 1,5, 15, 17, 19,21,23 | Combination of SEQ ID NOs: 2, 6, 16, 18, 20, 22, 24 | 27 and 28 |
| CL7 CAR | Combination of SEQ ID NOs: 1, 7, 15, 17, 19, 21, 23 | Combination of SEQ ID NOs: 2, 8, 16, 18, 20, 22, 24 | 29 and 30 |
| CL10 CAR | Combination of SEQ ID NOs: 1, 9, 15, 17, 19, 21, 23 | Combination of SEQ ID NOs: 2, 10, 16, 18, 20, 22, 24 | 31 and 32 |
| SL18 CAR | Combination of SEQ ID NOs: 1, 11, 15, 17, 19, 21, 23 | Combination of SEQ ID NOs: 2, 12, 16, 18, 20, 22, 24 | 33 and 34 |
| CD300c ECD CAR | Combination of SEQ ID NOs: 1, 13, 15, 17, 19, 21, 23 | Combination of SEQ ID NOs: 2, 14, 16, 18, 20, 22, 24 | 35 and 36 |

### Example 2. Preparation of recombinant lentivirus for expression of chimeric antigen receptor that specifically binds to CD300c antigen or receptor thereof

HEK293T cell line (ATCC) required for preparation of a recombinant lentivirus for expression of the chimeric antigen receptor that specifically binds to a CD300c antigen or a receptor thereof was prepared as follows. A complete medium used for cell culture was prepared by adding heat-treated fetal bovine serum (FBS) to fresh DMEM (Gibco) to a concentration of 10%, adding 1× Penicillin-Streptomycin (Gibco) thereto, and performing uniform mixing by inverting the resultant up and down. The resulting complete medium was preheated to 37°C and used. The HEK293T cell line was rapidly thawed for 2 to 3 minutes before use by quick transfer of its cryopreserved cell stock to a constant-temperature water bath at 37°C, inoculated into 30 mL of the complete medium, and cultured in a 5% CO₂ incubator at 37°C. When the cell confluency reached 80% or higher, the HEK293T cell line was maintained by subculture. For lentiviral transfection, the HEK293T cell line was inoculated into 10 mL of the complete medium at a concentration of 1 to 2 × 10⁶ cells, cultured for 16 hours, and then subjected to lentiviral transfection.

Lenti-X^{™} Expression System (Takara) kit was used for lentiviral transfection for expression of the chimeric antigen receptor that specifically binds to a CD300c antigen or a receptor thereof. To 7 µg of the expression vector pLVX-Puro/aCD300C scFv or pLVX-Puro/CD300c ECD-CAR constructed in the same manner as in Example 1 was added sterile water (Invitrogen) to make 600 µL. Then, the resultant was placed in a tube of Lenti-X Packaging Single Shots in Lenti-X^{™} Expression System and mixing was performed to prepare a nanoparticle complex solution. The nanoparticle complex solution was allowed to react at room temperature for 10 minutes, added dropwise to the previously prepared HEK293T cells, and then mixing was performed by shaking the resultant left and right. Culture was performed for 4 hours in an incubator, 6 mL of the fresh complete medium was further added, and cultured for 48 hours. After the culture, the supernatant was collected, centrifuged to remove cell debris, filtered through a 0.45 µm filter, and stored at -80°C until use.

20 µL of the obtained lentivirus supernatant was added to the sample well (S) of the GoStix cassette in Lenti-X^{™} Expression System, 3 drops of Chase solution were added to the sample well, and then reaction was allowed to occur at room temperature for 10 minutes. Subsequently, it was identified by presence or absence of a band whether a recombinant lentivirus at an effective dose of 5 × 10⁵ IFU/mL or higher was obtained.

As a result, it was identified that a recombinant lentivirus expressing the chimeric antigen receptor that specifically binds to a CD300c antigen or a receptor thereof was prepared. It was found that the prepared lentiviruses expressed chimeric antigen receptors, each of which comprises the amino acid sequence represented by SEQ ID NO: 4, 6, 8, 10, 12, or 14.

### Example 3: Production of Jurkat cells expressing chimeric antigen receptor that specifically binds to CD300c antigen or receptor thereof

To produce Jurkat cells expressing the chimeric antigen receptor that specifically binds to a CD300c antigen or a receptor thereof, the recombinant lentivirus prepared in the same manner as in Example 2 was transfected into a Jurkat cell line. More specifically, the recombinant lentivirus at 0.1 to 10 MOI was inoculated in the complete medium supplemented with polybrene (Merk) of 8 µg/mL, and uniform mixing was performed by inverting the resultant up and down. 1 mL of the mixture was respectively added to the Jurkat cell line prepared in a 6-well plate, centrifuged at 1,800 rpm for 45 to 90 minutes, and cultured at a 5% CO ₂ incubator at 37°C for 24 hours. 24 hours later, subculture was performed at a concentration of 5 × 10⁵ cells/mL.

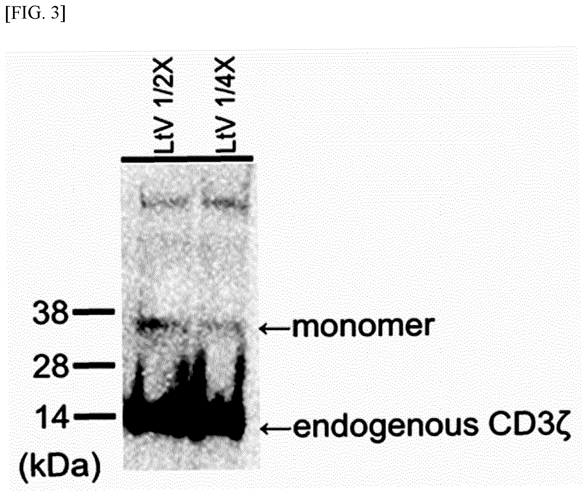

To check whether the chimeric antigen receptor that specifically binds to a CD300c antigen or a receptor thereof is normally expressed in the transfected Jurkat cell line, total proteins of the cultured Jurkat cells were obtained using PRO-PREP solution (iNtRON). Concentrations of the obtained proteins were measured using a microBCA protein assay kit (Thermo Fisher Scientific), and then Western blotting was performed using an equal amount of proteins. More specifically, the equal amount of proteins was subjected to electrophoresis by SDS-PAGE (Invitrogen), and the electrophoresed proteins were transferred to a nitrocellulose membrane (Invitrogen). The protein-bound nitrocellulose membrane was blocked using a 5% skim milk (BD) solution to block non-specific antibody reactions. An anti-CD3 antibody and an anti-GAPDH antibody (Cell Signaling Technologies, USA) as primary antibodies were respectively diluted to a concentration of 1:1,000 using 5% skim milk, and used to treat the nitrocellulose membrane. Reaction was allowed to occur, and then unbound antibodies were removed. A horseradish peroxidase (HRP)-conjugated secondary antibody (Cell Signaling Technologies) as a secondary antibody was diluted to a concentration of 1:2,000 and used to treat the nitrocellulose membrane. Then, treatment with ECL solution (Thermo Fisher Scientific) was performed to induce color development, and then the protein amount was quantified using an iBright 1500 luminescent image analyzer (Invitrogen). The results obtained by performing Western blotting are illustrated in FIG. 3.

As illustrated in FIG. 3, it was possible to identify the CD3ζ intracellular domain, to which the anti-CD3 antibody was bound. From these results, it was identified that a Jurkat cell line expressing the chimeric antigen receptor that specifically binds a CD300c antigen or a receptor thereof was produced.

Additionally, an expression level of the chimeric antigen receptor that specifically binds to a CD300c antigen or a receptor thereof was checked using a flow cytometer (FACS). More specifically, the Jurkat cell line was treated with a PE-fusion anti-IgG antibody (Jackson ImmunoResearch) and incubated at 4°C for 30 minutes to allow reaction to occur. Then, identification was performed using a flow cytometer.

As a result of flow cytometry, it was identified that a Jurkat cell line expressing the chimeric antigen receptor that specifically binds a CD300c antigen or a receptor thereof was produced.

### Example 4: Identification of expression of CD300c in cancer cells

To evaluate whether CD300c is expressed in various cancer cells, various human-derived solid cancer cell lines were cultured to evaluate the expression of CD300c at mRNA and protein levels. More specifically, A549, which is a human lung cancer cell line, MDA-MB-231, which is a human breast cancer cell line, and CT26, which is a mouse colorectal cancer cell line, were cultured, and each cell line was fixed with 4% formaldehyde. Then, blocking was performed with 5% normal goat serum. Treatment with 1 µg of the anti-CD300c antibody was performed and reaction was allowed to occur. Then, staining was performed with a FITC-labeled anti-rabbit IgG antibody. The fluorescently labeled cells were identified using a flow cytometer (FACS).

As a result, it was identified that the CD300c antigen is present on the cell surface of various cancers such as colorectal cancer, lung cancer, and breast cancer.

### Example 5: Anticancer effects of Jurkat cells expressing chimeric antigen receptor that specifically binds to CD300c antigen or receptor thereof

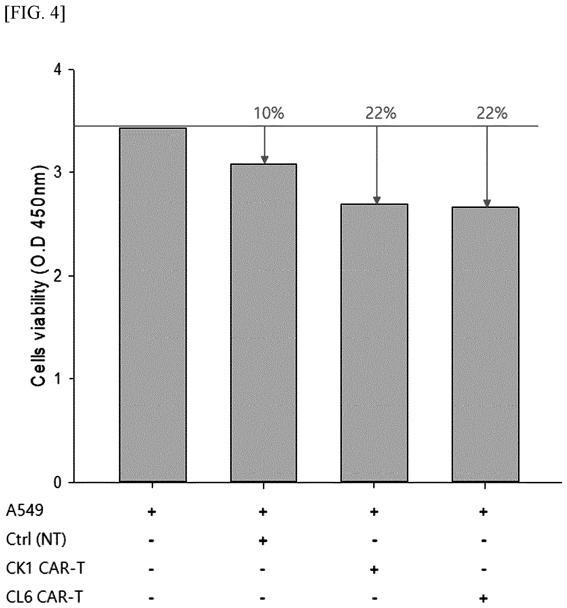

To identify anticancer effects of Jurkat cells expressing the chimeric antigen receptor that specifically binds to a CD300c antigen or a receptor thereof, their cancer cell killing effects were checked using A549 cell line. More specifically, the A549 cell line was inoculated into a 96-well plate at a concentration of 1 × 10⁵ cells/mL. Then, the Jurkat cells expressing the chimeric antigen receptor that specifically binds to a CD300c antigen or a receptor thereof, as produced in the same manner as in Example 3, were applied to the cancer cells to a concentration of 20:1 and co-culture was performed. Jurkat cells receiving no lentivirus transfection were used as a control. After co-culture for 24 hours, the co-cultured Jurkat cell line was removed from each well, and reaction was allowed to proceed for 1 hour using CCK-8 (DOJINDO). Then, the absorbance was measured at OD_{450 nm} to identify the degree of cancer cell death. The results are illustrated in FIG. 4.

As illustrated in FIG. 4, it was identified that the Jurkat cell line expressing the chimeric antigen receptor that specifically binds to a CD300c antigen or a receptor thereof exhibited anticancer effects against the A549 cell line, and these anticancer effects were higher than those of the Jurkat cell line receiving no lentivirus transfection.

From these results, it was found that use of the immune cell line expressing the chimeric antigen receptor that specifically binds to a CD300c antigen or a receptor thereof results in increased cancer therapeutic effects so that cancer can be effectively treated. In addition, it was found that the immune cell line specifically responds only to cancer cells expressing the CD300c antigen on the surface, and thus can exhibit maximized therapeutic effects with decreased adverse effects.

The description of the present disclosure as described above is provided for illustration, and those of ordinary skill in the art to which the present disclosure pertains will be able to understand that the embodiments disclosed herein can be easily modified into other specific forms without changing the technical spirit or essential features of the present disclosure. Therefore, it should be understood that the embodiments are illustrative and not restrictive in all respects.

### Industrial Applicability

The chimeric antigen receptor that specifically binds to a CD300c antigen or a receptor thereof of the present disclosure specifically binds to the CD300c antigen or a receptor thereof, and thus can be used for the treatment of all cancers that express the CD300c antigen. In addition, the chimeric antigen receptor can activate T cells and the like in the body through inhibition of CD300c so that decreased adverse effects and increased cancer therapeutic effects are achieved. Thus, the chimeric antigen receptor can be effectively used for immunotherapy of various cancers.

## Claims

1. A chimeric antigen receptor that specifically binds to a CD300c antigen or a receptor thereof, wherein the chimeric antigen receptor comprises any one or more selected from the group consisting of an antibody, a single domain antibody, and a single chain variable fragment, each of which specifically binds to the CD300c antigen or a receptor thereof, and an antigen.

2. The chimeric antigen receptor of claim 1, wherein the chimeric antigen receptor comprises the amino acid sequence represented by SEQ ID NO: 4, 6, 8, 10, 12, or 14.

3. The chimeric antigen receptor of claim 1, further comprising a signal peptide, a GS linker, a transmembrane domain, and an intracytoplasmic domain.

4. The chimeric antigen receptor of claim 3, wherein the signal peptide is a CD8α signal peptide.

5. The chimeric antigen receptor of claim 3, wherein the transmembrane domain is a CD8 hinge (hinge of cluster of differentiation 8) and a CD28 transmembrane domain.

6. The chimeric antigen receptor of claim 3, wherein the intracytoplasmic domain is a CD28 intracellular domain and a CD3ζ intracellular domain.

7. The chimeric antigen receptor of claim 1, wherein the chimeric antigen receptor is used for the treatment of cancer expressing the CD300c antigen or a CD300c receptor.

8. A recombinant vector that expresses the chimeric antigen receptor of any one of claims 1 to 7.

9. An immune cell transformed with the recombinant vector of claim 8.

10. The immune cell of claim 9, wherein the immune cell is at least one selected from the group consisting of monocytes, macrophages, T cells, natural killer cells (NK cells), and dendritic cells.

11. A pharmaceutical composition for preventing or treating cancer that expresses a CD300c antigen or a CD300c receptor, the pharmaceutical composition comprising the immune cells of claim 9 as an active ingredient.

12. The pharmaceutical composition of claim 11, wherein the cancer is any one or more selected from the group consisting of colorectal cancer, rectal cancer, colon cancer, thyroid cancer, oral cancer, pharyngeal cancer, laryngeal cancer, cervical cancer, brain cancer, lung cancer, ovarian cancer, bladder cancer, kidney cancer, liver cancer, pancreatic cancer, prostate cancer, skin cancer, tongue cancer, breast cancer, uterine cancer, stomach cancer, bone cancer, and blood cancer.

13. The pharmaceutical composition of claim 11, further comprising another cancer therapy.

14. The pharmaceutical composition of claim 11, wherein the pharmaceutical composition inhibits proliferation, survival, metastasis, recurrence, or cancer therapy resistance of cancer.

15. A method for preventing or treating cancer, comprising a step of administering to an individual a composition that comprises the immune cells of claim 9 as an active ingredient.

16. A use of a composition that comprises the immune cells of claim 9 as an active ingredient, for preventing or treating cancer.
